(19) Europäisches Patentamt
European Patent Office
Office européen des brevets

(11) EP 2 705 840 A1

(12) **EUROPEAN PATENT APPLICATION**

(43) Date of publication:
**12.03.2014 Bulletin 2014/11**

(21) Application number: **12306087.3**

(22) Date of filing: **10.09.2012**

(51) Int Cl.:
***A61K 31/133*** *(2006.01)* ***A61K 31/4409*** *(2006.01)*
***A61K 31/496*** *(2006.01)* ***A61K 31/4965*** *(2006.01)*
***A61K 31/513*** *(2006.01)* ***A61K 31/536*** *(2006.01)*
***A61K 31/675*** *(2006.01)* ***A61K 31/7036*** *(2006.01)*
***A61K 45/06*** *(2006.01)* ***A61P 31/06*** *(2006.01)*
***A61P 31/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventors:
- **Cieren-Puiseux, Isabelle**
  **75008 Paris (FR)**
- **Esposito, Virginie**
  **75008 Paris (FR)**
- **Farenc, Christine**
  **75008 Paris (FR)**
- **Maroni, Marilyn**
  **75008 Paris (FR)**
- **Perrin, Laurent**
  **75008 Paris (FR)**

(74) Representative: **Werner, Alain Henri**
**Sanofi**
**Département Brevets**
**54, rue La Boétie**
**75008 Paris (FR)**

(54) **Use of rifapentine in the treatment and/or prevention of active tuberculosis disease**

(57) The invention relates to new doses and regimens of rifapentine and use in the prevention/treatment of active tuberculosis disease or tuberculosis infection in patients infected with the human immunodeficiency virus (HIV) / acquired immune deficiency syndrome (AIDS) and treated with a combined antiretroviral combination, a combination of efavirenz, emtricitabine and tenofovir disoproxil fumarate.

EP 2 705 840 A1

## Description

The invention relates to new doses and regimens of rifapentine and use in the prevention and/or treatment of active tuberculosis disease or tuberculosis infection in patients infected with the human immunodeficiency virus (HIV) / acquired immune deficiency syndrome (AIDS) and treated with an antiretroviral combination, a combination of efavirenz, emtricitabine and tenofovir disoproxil fumarate.

Tuberculosis is a major cause of morbidity in HIV/AIDS patients.

Tuberculosis is an infectious bacterial disease caused by *Mycobacterium tuberculosis,* which most commonly affects the lungs. It is transmitted from person to person via droplets from the throat and lungs of people with the active respiratory disease.

About one-third of the world's population has latent TB, which means people have been infected by tuberculosis bacteria but are not (yet) ill with disease and cannot transmit the disease.

People infected with tuberculosis bacteria have a lifetime risk of falling ill with TB of 10%. However persons with compromised immune systems, such as people living with HIV, malnutrition or diabetes, or people who use tobacco, have a much higher risk of falling ill.

When a person develops active tuberculosis (disease), the symptoms (cough, fever, night sweats, weight loss etc.) may be mild for many months. This can lead to delays in seeking care, and results in transmission of the bacteria to others. People ill with TB can infect up to 10-15 other people through close contact over the course of a year. Without proper treatment up to two thirds of people ill with TB will die (WHO, 2012).

[State of the art]

Rifapentine in combination with an appropriate daily companion anti-tuberculosis drug (isoniazid, pyrazinamide and ethambutol or streptomycin) is today among the most effective active ingredients against *Mycobacterium tuberculosis.* However, rifapentine administered to population treated with antiretroviral combination represent a challenge due to potential interactions of rifamycins with antiretroviral combination.

Indeed, rifapentine is an inducer of CYP3A4 and is therefore expected to affect efavirenz pharmacokinetics via CYP induction after repeated administration. Rifapentine is also a moderate inhibitor of CYP3A4 and P-gp therefore modification of efavirenz exposure after single dose is likely. Then, rifapentine's acute inhibitory and chronic inductive drug interactions with efavirenz are anticipated.

There is still a need to find and optimize therapeutic approaches to treat HIV/AIDS positive patients coinfected with *Mycobacterium tuberculosis.*

The invention meets this need by providing new doses and regimens of rifapentine co-administrated with a combination of efavirenz, emtricitabine and tenofovir disoproxil fumarate.

Although in the context of active tuberculosis in which rifapentine daily dosing is envisaged for some months in HIV/AIDS patients, it has now been found, surprisingly, that new doses and regimens of rifapentine are compatible with a combination of efavirenz, emtricitabine and tenofovir disoproxil fumarate and do not exacerbate the toxicity of each drugs and which allows the appropriate prevention and/or treatment of tuberculosis disease or infection in HIV/AIDS patients.

[Brief description of the invention]

The invention relates to the use of new doses and regimens of rifapentine and/or combination containing rifapentine in the prevention and/or treatment of active tuberculosis disease or tuberculosis infection in HIV/AIDS patients treated with a combination of efavirenz, emtricitabine and tenofovir disoproxil fumarate.

Rifapentine, used usually as a free base, is administered in the form of 150 mg film coated tablets. The antiretroviral combination of 600 mg efavirenz, 300 mg emtricitabine and 200 mg tenofovir disoproxil fumarate is administered under the form of a fixed-dose combination of the three active substances.

[Definitions]

- Rifapentine is the International Nonproprietary Name (INN) of the compound corresponding to the chemical structure and chemical names:

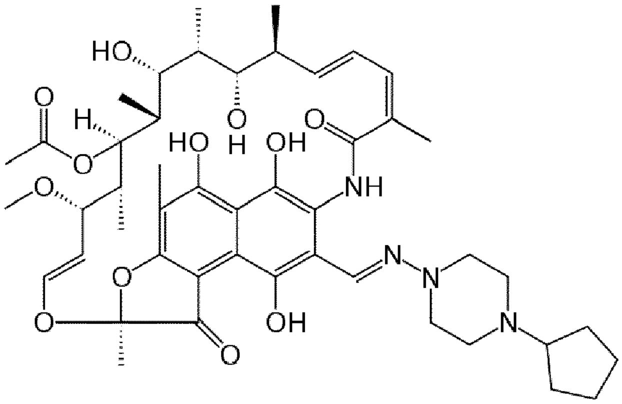

- 3-[[(4-cyclopentyl-1-piperazinyl)imino]methyl]- rifamycin, or
- 3-[N-(4-Cyclopentyl-1-piperazinyl)formimidoyl] rifamycin, or
- (7S,9E,11S,12R,13S,14R,15R,16R,17S,18S,19E,21Z,26E)-26-{[(4-cyclopentylpiperazin-1-yl)amino]methylidene}-2,15,17,29-tetrahydroxy-11-methoxy-3,7,12,14,16,18,22-heptamethyl-6,23,27-trioxo-8,30-dioxa-24-azatetracyclo[23.3.1.14,7.05,28]triaconta-1 (28),2,4,9,19,21,25(29)-heptaen-13-yl acetate.

Rifapentine is a rifamycin antimycobacterial indicated for the prevention and/or treatment of pulmonary tuberculosis caused by *Mycobacterium tuberculosis* in combination with one or more anti-tuberculosis companion drugs.

For treatment of pulmonary tuberculosis, the FDA-approved dose of rifapentine is 600 mg twice weekly for 2 months (intensive phase of tuberculosis treatment), with an interval of no less than 3 days (72 hours) between doses, as part of a regimen that includes appropriate daily anti-tuberculosis companion drugs that may include isoniazid, pyrazinamide and ethambutol or streptomycin.

- <u>Antiretroviral combination</u> is a combination of three active substances efavirenz, emtricitabine and tenofovir disoproxil fumarate. More particularly, the antiretroviral combination is a combination of 600 mg efavirenz, 300 mg emtricitabine and 200 mg tenofovir disoproxil fumarate given as a fixed dose combination. It is intended to provide an antiretroviral combination administered as a single, once-daily tablet for the treatment of HIV-1 infected adults. The mechanisms of action and the effects of efavirenz, emtricitabine and tenofovir disoproxil fumarate have been extensively investigated and are well-established. All three active substances are potent and selective inhibitors of HIV-1 reverse transcriptase (RT), weak inhibitors of human DNA polymerases, and show low potential to induce mitochondrial toxicity.

The combined use of these three substances is recommended in national and international HIV-1 infection treatment guidelines (e.g. national European guidelines, US guidelines and WHO guidelines) due to a lack of cross-resistance, dual antagonism and significant overlap in toxicities, respectively. The fixed combination hence aims to simplify regimens and to improve adherence to the combination.

- <u>HIV/AIDS patients under the antiretroviral combination</u> are HIV infected patients with a CD4 count cells of at least 400 receiving a fixed dose combination of 600mg efavirenz/300mg emtricitabine/200mg tenofovir disoproxil fumarate.

[Description of the invention]

The present invention relates to the use of new doses and regimens of rifapentine in the preparation of medicament intended for the prevention and/or treatment of active tuberculosis disease or tuberculosis infection in HIV/AIDS infected patients treated with an antiretroviral combination.

Indeed, the present invention relates to the use of rifapentine for use in the prevention and/or treatment of active tuberculosis disease or tuberculosis infection in HIV/AIDS infected patients treated with an antiretroviral combination.

For pulmonary tuberculosis, the Food and Drug Administration approved dose of rifapentine is 600 mg twice weekly for two months (intensive phase for tuberculosis treatment) with an interval of no less than 3 days (72 hours) between doses. In this interaction study, rifapentine may be administered at three different dosages and regimen:

1. 15 mg/kg once a day;
2. 10 mg/kg twice a day;

3. weekly administrations of 900 mg of rifapentine.

The invention also relates to a dosage and regimen where the rifapentine is administered once a day during the treatment period.

The invention also relates to a dosage and regimen where the rifapentine is administered at 15 mg/kg once a day during the treatment period.

The invention also relates to a dosage and regimen where the rifapentine is administered twice a day during the treatment period.

The invention also relates to a dosage and regimen where the rifapentine is administered at 10 mg/kg twice a day during the treatment period.

The invention also relates to dosage and regimen where the rifapentine is administered once a week during the treatment period.

The invention also relates to dosage and regimen where the rifapentine is administered at 900 mg once a week during the treatment period.

The invention also concerns a method of treatment of active tuberculosis disease or tuberculosis infection in HIV/AIDS infected patients in need and treated with a combination of 600mg efavirenz, 300mg emtricitabine and 200mg tenofovir disoproxil fumarate thereof, said method comprising the administration to said patient therapeutically effective doses of rifapentine according to the invention.

The invention also relates to a pharmaceutical composition containing rifapentine for use as a compound in the treatment of active tuberculosis disease or tuberculosis infection in HIV/AIDS patients treated with an antiretroviral combination.

Thus, another subject-matter of the present invention is a pharmaceutical composition comprising doses and regimens of rifapentine according to the present invention in combination with one (or more) active principle(s) chosen from one of the following appropriate daily companion anti-tuberculosis drugs that may include isoniazid, pyrazinamide and ethambutol or streptomycin.

The invention also relates to a pharmaceutical kit, which comprises:

(i) a first pharmaceutical composition comprising rifapentine, possibly in combination with an appropriate daily companion anti-tuberculosis drug;

(ii) a second galenic formulation comprising a combination of efavirenz, emtricitabine and tenofovir;

both pharmaceutical compositions (i) and (ii) being intended to be independently administered, each administration with regard to the other one being simultaneous, separated or spread over the time.

The invention also relates to the above pharmaceutical kit for use in the prevention and/or treatment of active tuberculosis disease or tuberculosis infection in HIV/AIDS patients treated with a combination of efavirenz, emtricitabine and tenofovir disoproxil fumarate.

The HIV/AIDS infected under treatment with an antiretroviral combination belong to the population with:

- HIV positive with CD4 count cells of at least 400 and receiving antiretroviral combination,
- Anti-human immunodeficiency virus 1 and 2 antibodies (anti-HIV1 or anti HIV2 Ab) test positive documented,
- Negative quantiferon test + Chest X-Rays normal and culture negative results from sputa for M. tuberculosis (enrolment is allowed before tuberculosis culture data are available/ patients would be withdrawn after enrolment if cultures turn out positive).

The evaluation of the effect of single and repeated administration of rifapentine and the antiretroviral combination is done repeatedly over a period of 21 days or three weeks according to different regimens (daily or weekly regimen) of rifapentine.

Examples of regimen and doses are given in the study below.

An open-label, non-randomized, single sequence, two periods, four-treatment, three parallel groups pharmacokinetic study of repeated oral doses (daily or weekly regimen) of rifapentine on the antiretroviral combination given to HIV/AIDS positive patients is disclosed below.

<u>**STUDY OBJECTIVE(S)**</u>

**Primary:**

To evaluate the effect of single and repeated administration of rifapentine given as daily or weekly regimen on steady-state PK parameters of efavirenz, emtricitabine and tenofovir given as a fixed dose combination (ATRIPLA™).

**Secondary:**

To evaluate the safety and tolerability of concomitant administration of rifapentine and the antiretroviral combination given to HIV+ patients.

**STUDY DESIGN**

This study is an open label, non-randomized, single sequence (Period 1 followed by Period 2), two periods (Period 1: antiretroviral combination - Period 2: antiretroviral combination co-administered with rifapentine, four treatments antiretroviral combination, rifapentine daily regimen of 15mg/kg once a day (OD) or 10 mg/kg twice a day (BID) or rifapentine weekly regimen of 900 mg) study performed in three parallel cohorts as follow (FIGURE 1):

- 1 st cohort:

    o Period 1: patients will receive a daily dose of antiretroviral combination during 15 days,
    o Period 2: patients will receive a daily dose of antiretroviral combination and 15mg/kg of oral rifapentine once a day (OD) during 21 days.

- 2nd cohort:

    o Period 1: patients will receive a daily dose of antiretroviral combination during 15 days,
    o Period 2: patients will receive a daily dose of antiretroviral combination and 900mg of oral rifapentine (once weekly - 3 weekly administrations).

- 3rd cohort (optional):

    o Period 1: patients will receive a daily dose of antiretroviral combination during 15 days,
    o Period 2: patients will receive a daily dose of antiretroviral combination and 10mg/kg of oral rifapentine twice a day (BID) during 21 days.

Patients who will be enrolled in this study should be on antiretroviral combination before the screening start (background therapy) and administered the same dose and regimen during the all study duration.

Both cohorts 1 and 2 will be started first in parallel. The last cohort 3 is optional and may be not performed depending on the availability of safety and relevance of pharmacokinetic data from the ongoing Phase 1 study during the course of the present study.

**STUDY POPULATION**

Main selection criteria

**Inclusion criteria:**

- Age 18 to 55 years old inclusive.
- HIV positive with CD4 count cells of at least 400 and receiving antiretroviral combination;
- Anti-human immunodeficiency virus 1 and 2 antibodies (anti-HIV1 or anti HIV2 Ab) test positive documented.
- Negative quantiferon test + Chest X-Rays normal and culture negative results from sputa for *M. tuberculosis* (enrolment is allowed before tuberculosis culture data are available/patients would be withdrawn after enrolment if cultures turn out positive).
- Female subjects not post-menopausal or not surgically sterile not using effective contraception.

**Exclusion criteria:**

• Age < 18 years old.

*Related to the methodology*

- Any medication (including St John's Wort) within 14 days before inclusion or within 5 times the elimination half-life or pharmacodynamic half-life of the medication, with the exception of a combination of a combination of 600mg efavirenz, 300mg emtricitabine and 200mg tenofovir disoproxil fumarate, multivitamins, acetaminophen (up to 650 mg every 6 hours as an analgesic), ibuprofen (up to 600 mg twice daily), naproxen (up to 500 mg twice daily for pain or headache) are permitted.
- Any vaccination within the last 28 days.

*Related to the disease*

- Positive result on hepatitis B surface (HBs Ag) antigen. Patients with anti-hepatitis C virus (anti-HCV) antibodies can be enrolled if they have a history of HCV-PCR negative within 1 year.
- Positive result on urine drug screen (amphetamines/methamphetamines, barbiturates, benzodiazepines, cannabinoids, cocaine, opiates).
- Positive alcohol test.

*Related to the health status*

- Any history or presence of clinically relevant cardiovascular, pulmonary, gastrointestinal, hepatic, renal, metabolic, haematological (patients with porphyria), neurological, osteomuscular, articular, psychiatric, systemic, ocular, gynaecologic (if female), or infectious disease, or signs of acute illness other than HIV disease.
- Frequent headaches and/or migraine, recurrent nausea and/or vomiting (more than twice a month).
- Blood donation, any volume, within 2 months before inclusion.
- Symptomatic postural hypotension, irrespective of the decrease in blood pressure, or asymptomatic postural hypotension defined as a decrease in systolic blood pressure ≥20 mmHg within 3 minutes when changing from supine to standing position.
- Presence or history of drug hypersensitivity, or allergic disease diagnosed and treated by a physician.
- History or presence of drug or alcohol abuse (alcohol consumption more than 40 g per day).
- Excessive consumption of beverages containing xanthine bases (more than 4 cups or glasses per day).

<u>**INVESTIGATIONAL PRODUCT(S)**</u>

Rifapentine: 150mg film-coated tablet in fed conditions in the morning (cohort 1 and cohort 2) or morning and evening (cohort 3).

Antiretroviral combination: fixed dose combination of 600mg efavirenz/300mg emtricitabine/200mg tenofovir disoproxil fumarate: 1 tablet (commercial formulation: ATRIPLA™) taken at least 2 hours after the evening meal (fasting condition) once daily and at night (bedtime).

<u>**Dose regimen**</u>

The following dose levels are planned:

**Rifapentine:**

- <u>Daily regimen:</u>

o 15mg/kg once daily dosing of oral rifapentine in fed conditions in the morning during 21 days (**cohort 1**).

| Weight Range (kg) | total Daily Dose (mg) | Number of Rifapentine Tablets to Administer per dose |
|---|---|---|
| 40-49.9 | 600 | 4 |

(continued)

| Weight Range (kg) | total Daily Dose (mg) | Number of Rifapentine Tablets to Administer per dose |
|---|---|---|
| 50-69.9 | 900 | 6 |
| 70-89.9 | 1200 | 8 |
| 90-99.9 | 1500 | 10 |

o 10mg/kg BID (morning and evening) in fed conditions during 21 days. On Day 1, rifapentine is given on the evening only (single dose) (**cohort 3**).

| Weight Range (kg) | **Give this dose once daily (mg)** | Number of Rifapentine Tablets to Administer per dose | Number of Rifapentine Tablets to Administer per day |
|---|---|---|---|
| 40-49.9 | 450 | 3 | 6 |
| 50-69.9 | 600 | 4 | 8 |
| 70-79.9 | 750 | 5 | 10 |
| 80-99.9 | 900 | 6 | 12 |

- Weekly regimen:

o 900mg once weekly (3 weekly administrations) in fed conditions in the morning (**cohort 2**).

**ATRIPLA™**:

- Daily Regimen: one tablet once daily, at least 2 hours after the evening meal (fasting conditions) and at night (bedtime).

PRIMARY ENDPOINT(S) AND MAIN SECONDARY ENDPOINT(S)

**Primary:**

- **Pharmacokinetic parameters:** (Cmax, Cmin and AUC0-24 for antiretroviral combination on Day-2, Day 1 and Day 21 for cohorts 1 and 3 & on Day -2, Day 1 and Day 16 for cohort 2).

**Secondary (main):**

- **Pharmacokinetic parameters:**
    - $t_{1/2z}$, $t_{max}$, $t_{lag}$, CL/F for antiretroviral combination on Day-2, Day 1 and Day 21 for cohorts 1 and 3 & on Day -2 ,Day 1 and Day 16 for cohort 2,
    - $AUC_{0}$-$_{10}$ for antiretroviral combination on Day -2 and Day 1 for cohorts 1 and 3,
    - $C_{trough}$ and $C_{8h}$ (for cohort 2 only) for rifapentine and 25-desacetyl- rifapentine (25-DR) on Day 1, 8, and 15.
- **Safety parameters:** The tolerability investigations at baseline and during the study will consist of:

**Physical examinations**

Heart and respiratory auscultation

- peripheral arterial pulse,
- pupil, knee, Achilles plantar reflexes,
- *peripheral lymph nodes and abdomen examination.

Vital signs

Heart rate, systolic and diastolic blood pressure measured after 10 minutes in supine resting position and after 3 minutes in standing position.

**Adverse events**

Hematology

- red blood cell count,

- hematocrit, hemoglobin,

- white blood cell count with differential count (neutrophils, eosinophils, basophils, monocytes, and lymphocytes).

**Coagulation parameters**

Serum chemistry

- Urinalysis proteins, glucose, erythrocytes, leucocytes, ketone bodies, and pH,
- CD4 cells count,
- viral load monitoring.

**PHARMACOKINETICS**

Blood samples for pharmacokinetic analyses of the two compounds will be obtained from all patients as follow.

Combination of efavirenz, emtricitabine and tenofovir disoproxil fumarate

- Cohort 1 and 3

Blood samples will be collected at predose, 0.5, 0.75, 1, 1.5, 2, 3, 4, 5, 6, 8, 10, 16, and 24 hours post-dose of the antiretroviral combination on Day -2, Day 1 and Day 21.

- Cohort 2

Blood samples will be collected at predose, 0.5, 0.75, 1, 1.5, 2, 3, 4, 5, 6, 8, 10, 16, and 24 hours post-dose of antiretroviral combination on Day -2, Day 1 and Day 16.

Rifapentine and 25-Desacetyl Rifapentine (active metabolite):

- Once daily regimen (cohort 1)

Blood samples are collected at pre-dose on Day 1, 5; 8, 15, 18, 21 and 24h after last dosing.

• BID regimen (cohort 3)

Blood samples are collected at pre-dose on Day 1, then predose of 1st dosing on Day 5, 8, 15, 18, 21 and 14h after last dosing.

• Weekly regimen (cohort 2)

Blood samples are collected at predose on Day 1 and 8H post dose on Day 1, 8, and 15.

[PHARMACOGENETIC SAMPLES]

At the study visit as specified in the study flow chart (Day -2), a blood sample will be collected to investigate allelic variants of drug metabolism enzymes or drug transporters in particular CYP2B6 which is involved in the clearance of efavirenz and OATPB1 which is expected to be involved in the clearance of rifapentine.

STATISTICAL CONSIDERATIONS

The primary objective of the trial is to evaluate the effect of single and repeated administration of rifapentine given as daily or weekly regimen on steady-state PK parameters of efavirenz, emtricitabine and tenofovir disoproxil fumarate given as a fixed dose combination.
All the statistical analyses will be done separately for each cohort.

Effect of rifapentine (repeated dose) on the antiretroviral combination at steady state

• Descriptive statistics

Pharmacokinetic parameters of the antiretroviral combination will be summarized using descriptive statistics by cohort, treatment group (efavirenz alone, emtricitabine alone, tenofovir disoproxil fumarate alone, efavirenz + rifapentine, emtricitabine + rifapentine, tenofovir disoproxil fumarate + rifapentine), day (Day-2 for the combination of the three active drugs regarding all cohorts, Day 16 for cohort 2 and Day 21 for cohort 1 and 3 regarding the coadministration combination of the three active drugs + rifapentine) after combination of the three active drugs alone and after coadministration with rifapentine.
Treatment ratios of efavirenz (respectively emtricitabine, tenofovir) coadministered with rifapentine versus efavirenz (resp. emtricitabine, tenofovir) alone for Cmax, Cmin, AUC0-10 and t1/2z of efavirenz (resp. emtricitabine, tenofovir) will be listed by cohort, patient, gender, day and summarized using descriptive statistics.

• Inferential analysis

Prior to the analyses described below, Cmax, Cmin, AUC0-24 and t1/2z will be log-transformed. These parameters will be analyzed separately for each cohort using the following linear mixed effects model:

Log(Parameter) = Treatment + Gender + Error with fixed terms for treatment (combination

of the three active drugs alone, combination of the three active drugs co-administered with rifapentine) and gender, and with an unstructured 2 by 2 matrix of treatment-specific variances and covariances for subject-within-gender using SAS Proc Mixed®. In case of convergence problems, other variance-covariance structures will be explored.
For Cmax, Cmin, AUC0-24 and t1/2z, estimates and 90% confidence intervals (CIs) for the geometric mean ratio of efavirenz (respectively emtricitabine, tenofovir) coadministered with rifapentine versus efavirenz (resp. emtricitabine, tenofovir) alone will be obtained by computing estimates and 90% CIs for the differences between treatment means within the linear mixed effects model framework, and then converting to ratios by the antilog transformation.

• tmax

For each cohort, the distribution of tmax values will be represented by histogram plots separately for efavirenz (respectively emtricitabine, tenofovir) alone and efavirenz (respectively emtricitabine, tenofovir) coadministered with rifapentine. In addition histogram of differences in tmax between efavirenz (respectively emtricitabine, tenofovir) when

coadministered with rifapentine versus efavirenz (respectively emtricitabine, tenofovir) alone will be provided.

• Variance component

For each cohort separately, within-subject and total standard deviations will be estimated for log-transformed Cmax, Cmin and AUC0-24 by using data at Day-2 (antiretroviral combination alone), Day 21 (combination of the three active drugs + rifapentine for cohorts 1 and 3), Day 16 (combination of the three active drugs+rifapentine for cohort 2) and by equating observed and expected mean squares within the linear mixed effects model framework with a homogeneous variance components structure. 90% confidence intervals will be computed using the simple $X^2$ method for within-subject SD and the Graybill-Wang procedure for the total SD (Burdick RK, Graybill FA. Confidence Intervals on Variance Components. New York: Marcel Dekker, Inc; 1992).

DURATION OF STUDY PERIOD

(62 days per Patient at maximum)

The duration of the study for each patient will include:

- An up to 21-day screening phase,
- Treatment period(s): the study observation period starts at period 1 and lasts until the end of the study visit. In Period 1, patients will receive a combination of the three active drugs (unchanged and stable treatment since the screening) and in Period 2 co-administration of rifapentine and a combination of the three active drugs for 3 weeks (21 days),
- End of study: patients will be followed 3 to 5 days (end-of-study visit) after the last administration of rifapentine while the combination of the three active drugs will be continued. However, patient participation could be prolonged in case of safety concerns,
- Total duration from screening per patient: 62 days at maximum.

## Claims

1. Rifapentine for use in the prevention and/or treatment of active tuberculosis disease or tuberculosis infection in HIV/AIDS infected patients treated with an antiretroviral combination.

2. Rifapentine for use according to claim 1 where the use does not exacerbate the toxicity of each drugs and which allows the appropriate prevention and/or treatment of tuberculosis disease or infection in HIV/AIDS patients.

3. Rifapentine for use according to any one of claims 1 or 2 where the antiretroviral combination is a combination of efavirenz, emtricitabine and tenofovir disoproxil fumarate.

4. Rifapentine for use according to claim 1 where the antiretroviral combination is a combination of 600 mg efavirenz, 300 mg of emtricitabine and 200 mg tenofovir disoproxil fumarate.

5. Rifapentine for use according to any one of claims 1 to 4 where rifapentine is administered once a day during the treatment period.

6. Rifapentine for use according to claim 5 where rifapentine is administered at a dosage of 15 mg/kg once a day during the treatment period.

7. Rifapentine for use according to any one of claims 1 to 3 where the rifapentine is administered twice a day during the treatment period.

8. Rifapentine for use according to claim 7 where rifapentine is administered at a dosage of 10 mg/kg twice a day during the treatment period.

9. Rifapentine for use according to any one of claims 1 to 3 where the rifapentine is administered once a week during the treatment period.

10. Rifapentine for use according to claim 9 where the rifapentine is administered at a dosage of 900 mg once a week

during the treatment period.

**11.** Rifapentine for use according to claim 1 to 10 where the HIV/AIDS infected patients treated with an antiretroviral combination are:

- Age 18 to 55 years old inclusive,
- HIV positive with CD4 count cells of at least 400 and receiving antiretroviral combination,
- Anti-human immunodeficiency virus 1 and 2 antibodies (anti-HIV1 or anti HIV2 Ab) test positive documented,
- Negative quantiferon test + Chest X-Rays normal and culture negative results from sputa for *M. tuberculosis* (enrolment is allowed before tuberculosis culture data are available/ patients would be withdrawn after enrolment if cultures turn out positive).

**12.** Pharmaceutical composition comprising rifapentine for use according to any one of claims 1 to 11.

**13.** Pharmaceutical composition according to claim 12 comprising in addition to rifapentine one or more appropriate daily companion anti-tuberculosis drugs.

**14.** Rifapentine and one or more appropriate daily companion anti-tuberculosis drugs for use in the treatment of active tuberculosis disease or tuberculosis infection in HIV/AIDS infected patients treated with an antiretroviral combination.

**15.** Pharmaceutical composition according to claim 13 wherein the appropriate daily companion anti-tuberculosis drugs are chosen among isoniazid, pyrazinamide and ethambutol or streptomycin.

**16.** A pharmaceutical kit, which comprises:

(i) a first pharmaceutical composition comprising rifapentine for use according to claim 1 to 11, possibly in combination with an appropriate daily companion anti-tuberculosis drug;
(ii) a second galenic formulation comprising a combination of efavirenz, emtricitabine and tenofovir;
both pharmaceutical compositions (i) and (ii) being intended to be independently administered, each administration with regard to the other one being simultaneous, separated or spread over the time.

**17.** Use of rifapentine being in the preparation of medicament intended for the prevention and/or treatment of active tuberculosis disease or tuberculosis infection in HIV/AIDS infected patients treated with a combined antiretroviral combination.

FIGURE 1

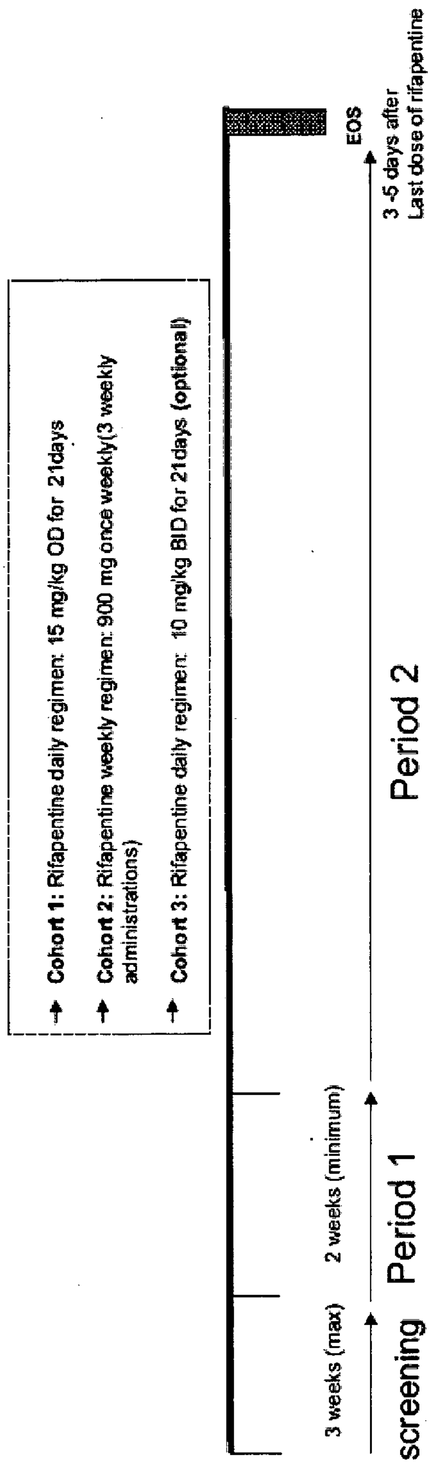
Cohort 1: Rifapentine daily regimen: 15 mg/kg OD for 21days
Cohort 2: Rifapentine weekly regimen: 900 mg once weekly(3 weekly administrations)
Cohort 3: Rifapentine daily regimen: 10 mg/kg BID for 21days (optional)
3 weeks (max)
2 weeks (minimum)
screening
Period 1
Period 2
EOS
3 -5 days after
Last dose of rifapentine

Europäisches Patentamt
European Patent Office
Office européen des brevets

# EUROPEAN SEARCH REPORT

Application Number
EP 12 30 6087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Dorman Susan et al.: "Rifapentine no better than rifampin for intensive phase treatment of pulmonary TB", EATG , 15 August 2012 (2012-08-15), XP002691685, Retrieved from the Internet: URL:http://old.eatg.org/eatg/Global-HIV-News/TB-Malaria/Rifapentine-no-better-than-rifampin-for-intensive-phase-treatment-of-pulmonary-TB [retrieved on 2013-02-05] * abstract * | 1,2,5,9, 12-15,17 | INV. A61K31/133 A61K31/4409 A61K31/496 A61K31/4965 A61K31/513 A61K31/536 A61K31/675 A61K31/7036 A61K45/06 A61P31/06 A61P31/18 |
| X | DORMAN SUSAN E ET AL: "Substitution of rifapentine for rifampin during intensive phase treatment of pulmonary tuberculosis: study 29 of the tuberculosis trials consortium.", THE JOURNAL OF INFECTIOUS DISEASES, vol. 206, no. 7, 30 July 2012 (2012-07-30) , pages 1030-1040, XP002691686, ISSN: 1537-6613 * abstract * * page 1036, right-hand column * * page 1035 - page 1036; tables 3,4 * | 1,2,5,8, 12-15,17 | TECHNICAL FIELDS SEARCHED (IPC) A61K |
| X | POTTER BETH ET AL: "Management of active tuberculosis.", AMERICAN FAMILY PHYSICIAN, vol. 72, no. 11, 1 December 2005 (2005-12-01), pages 2225-2232, XP002691687, ISSN: 0002-838X * page 2228; table 3 * * page 2230; table 4 * | 1,2,9, 12-15,17 | |

-/--

The present search report has been drawn up for all claims

| Place of search | Date of completion of the search | Examiner |
|---|---|---|
| The Hague | 15 May 2013 | Opravz, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

Europäisches Patentamt
European Patent Office
Office européen des brevets

# EUROPEAN SEARCH REPORT

Application Number
EP 12 30 6087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOCK NAOMI N ET AL: "A prospective, randomized, double-blind study of the tolerability of rifapentine 600, 900, and 1,200 mg plus isoniazid in the continuation phase of tuberculosis treatment.", AMERICAN JOURNAL OF RESPIRATORY AND CRITICAL CARE MEDICINE, vol. 165, no. 11, 1 June 2002 (2002-06-01), pages 1526-1530, XP002691688, ISSN: 1073-449X * page 1526 * * page 1527; table 1 * ----- | 1,2,9, 10, 12-15,17 | |
| X | Anonymous: "TBTC Study 29: Rifapentine During Intensive Phase Tuberculosis (TB) Treatment", U.S. National Institutes of Health , 7 May 2012 (2012-05-07), XP002691689, Retrieved from the Internet: URL:http://clinicaltrials.gov/show/NCT00694629 [retrieved on 2013-02-06] * the whole document * ----- | 1,2,5,6, 12-15,17 | TECHNICAL FIELDS SEARCHED (IPC) |

-/--

The present search report has been drawn up for all claims

| Place of search | Date of completion of the search | Examiner |
|---|---|---|
| The Hague | 15 May 2013 | Opravz, Petra |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | jameszhang5: "After BCG treatment, my father has suffered bladder and Urinary tract pain (8 month)", Better Medicine Forum , April 2008 (2008-04), XP002691690, Retrieved from the Internet: URL:http://forums.bettermedicine.com/showthread.php/28107-after-bcg-treatment-my-father-has-suffered-bladder-and-urinary-tract-pain-8-month [retrieved on 2013-02-06] * abstract * ----- | 1,2,7, 12-15,17 | |
| X | Anonymous: "Rifapentine", Pipeline Report , 17 July 2012 (2012-07-17), XP002697043, Retrieved from the Internet: URL:http://www.pipelinereport.org/browse/tb-treatments/rifapentine [retrieved on 2013-05-14] * abstract * ----- | 1-17 | |
| | | | TECHNICAL FIELDS SEARCHED (IPC) |

The present search report has been drawn up for all claims

3

| Place of search | Date of completion of the search | Examiner |
|---|---|---|
| The Hague | 15 May 2013 | Opravz, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 12 30 6087

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 12 30 6087

The Search Division considers that the present European patent application does not comply with the requirements of unity of invention and relates to several inventions or groups of inventions, namely:

1. claims: 1, 2, 5-10, 12-15, 17(all partially)

   Rifapentine for use in the prevention and/or treatment of active tuberculosis disease.

   ---

2. claims: 3, 4, 11, 16(completely); 1, 2, 5-10, 12-15, 17(partially)

   Rifapentine for use in the prevention and/or treatment of tuberculosis infection in HIV/AIDS infected patients treated with an antiretroviral combination.

   ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BURDICK RK ; GRAYBILL FA.** Confidence Intervals on Variance Components. Marcel Dekker, Inc, 1992